# EUROPEAN PATENT APPLICATION

(11) **EP 4 339 616 A1**
(43) Date of publication of application: **20.03.2024**
(21) Application number: 22816433.1
(22) Date of filing: 31.05.2022
(51) Int. Cl.: G01N 33/574, G01N 33/92, G01N 30/72

(54) **BIOMARKER COMPOSITION CONTAINING ACYL CARNITINE METABOLITE FOR DIAGNOSIS OF ORAL CANCER**

(30) Priority: 01.06.2021 KR 20210070830
(71) Applicant: National Cancer Center, Goyang-si, Gyeonggi-do 63243 (KR); Korea Basic Science Institute, Daejeon 34133 (KR)
(72) Inventor: KIM, Mi Kyung, Goyang-si Gyeonggi-do 10324 (KR); CHOI, Sung Weon, Seoul 06511 (KR); HWANG, Geum Sook, Seoul 06798 (KR); JUNG, Young Ae, Seoul 08701 (KR)
(74) Representative: Isarpatent
(86) International application number: PCT/KR2022/007731
(87) International publication number: WO 2022/255774

(57) **Abstract**

The present disclosure relates to a biomarker for diagnosis of oral cancer, using metabolite profiling and, more specifically, to a biomarker composition containing an acyl carnitine metabolite which varies in quantity upon the onset of oral cancer, and a diagnostic method for oral cancer, using same. The present disclosure allows for the early diagnosis of oral cancer which has been conventionally difficult to diagnose due to the impossible tissue biopsy thereof, and can reduce the pain and risk burden of patients because it uses a liquid biopsy method in which a blood sample is collected and analyzed.

## Description

### TECHNICAL FIELD

### CROSS-REFERENCE TO RELATED APPLICATIONS

This application is a National Stage of International Application No. PCT/KR2022/007731 filed May 31, 2022, claiming priority based on Korean Patent Application No. 10-2021-0070830, filed on June 1, 2020.

The present disclosure relates to a biomarker for diagnosing oral cancer using metabolomics profiling, and more particularly, to a biomarker composition including acyl carnitine metabolites, the amount of which changes when oral cancer occurs, and a method for diagnosing oral cancer using the same.

### BACKGROUND ART

Oral cancer is one of the most common malignant tumors in the world. According to the World Health Organization (WHO) GLOBOCAN data, there were 378,000 new cases of oral cancer diagnosed worldwide in 2020, and this number is projected to increase to 553,000 by 2040. In 2020, there were 178,000 deaths from oral cancer, and this number is projected to increase to 263,000 by 2040. In terms of types of oral caners, more than 90% of oral cancers are oral squamous cell carcinomas (OSCC). The overall 5-year survival rate for oral cancer is approximately 56%, which is a low survival rate among cancer types. Researchers have published various results on treatment and reconstruction methods. In spite of many advances in diagnosis and treatment methods, the survival rates of oral and oropharyngeal cancer have not increased significantly over the past few decades.

Oral cancer is often asymptomatic in the early stages and there are few specific diagnostic methods, so that it may be significantly difficult to detect oral cancer in the early stage. Most cases of oral cancers are diagnosed in advanced stages, resulting in low survival rates and poor prognosis. Therefore, it is significantly important to find non-invasive and highly sensitive biomarkers, capable of diagnosing oral cancers as early as possible, and to use the found biomarkers in diagnosis.

Metabolomics is a recent field of technology that follows genomics, transcriptomics, and proteomics, and is a branch of biology that comprehensively analyzes and studies metabolites and metabolic pathways in cells. Metabolomics is an important research field for studying metabolic processes in the body, identifying important biomarkers related to metabolic characteristics, and elucidating metabolic mechanisms. Metabolomics includes the identification, profiling, and quantification of metabolites, which are the end products of biological processes that play an important role in linking genotype to phenotype. In particular, in the field of cancer research, metabolomics is expected to play an important role in the strategy of cancer treatment using metabolic anticancer drugs by finding cancer metabolites that change during the carcinogenesis process and elucidating how the changes in the metabolites contribute to specific genes or mechanisms.

The most common metabolomic analysis platforms used to comprehensively analyze and quantify metabolites in biological systems are nuclear magnetic resonance (NMR) spectroscopy and mass spectrometry (MS), which are each coupled with chromatography. Metabolomics analysis techniques based on such platforms have been effectively and widely used to identify biomarkers for diagnosis and etiological investigation of various cancers, such as breast cancer, colorectal cancer, lung cancer, prostate cancer, or hepatocellular carcinoma.

While various studies related to oral cancer have been reported, only few studies are highly reliable and clinically validated. For this reason, metabolites have not yet been widely used as clinical markers for screening or detecting oral cancer. In addition, there is a need for research to find biomarkers using non-invasive or liquid biopsy, applicable to actual clinical practices and examinations.

The present inventors performed targeted and non-targeted metabolomics profiling based on UHPLC-ESI-Q-TOF-MS/MS from an oral cancer patient group and a control group to confirm that acyl carnitine metabolites may be used as useful metabolic signals for identifying oral cancer.

### PRIOR ART DOCUMENTS

### Patent Documents

1. Korean Patent No. 10-2091483

### Non- Patent Documents

1. Martin-Blαzquez A, et al. Untargeted LC-HRMS-based metabolomics to identify novel biomarkers of metastatic colorectal cancer. Scientific reports 9, 20198 (2019).
2. Xie GX, et al. Urine metabolite profiling offers potential early diagnosis of oral cancer. Metabolomics 8, 220-231 (2012).
3. Chen X, Yu D. Metabolomics study of oral cancers. Metabolomics 15, 22 (2019).

### DISCLOSURE OF THE INVENTION

### TECHNICAL PROBLEM

An object of the present disclosure is to provide a biomarker composition for diagnosing oral cancer that includes acyl carnitine metabolites as effective ingredients, a composition for diagnosing oral cancer that includes an agent for measuring the level of acyl carnitine metabolites as an effective ingredient, a kit for diagnosing oral cancer that includes the composition for diagnosing oral cancer, and a method of providing information for diagnosing oral cancer.

### TECHNICAL SOLUTION

According to an embodiment of the disclosure, a biomarker composition for diagnosis of oral cancer comprises an acyl carnitine metabolite as an effective ingredient.

The acyl carnitine metabolite may comprise at least one selected from the group consisting of decanoylcarnitine, octanoylcarnitine, and hexanoylcarnitine.

The biomarker composition for diagnosis of oral cancer may further comprises at least one metabolite selected from a group consisting of glycerophosphorylcholine, arachidonic acid, eicosapentaenoic acid, and triglyceride.

Further, according to an embodiment of the disclosure, a composition for diagnosis of oral cancer, comprising an agent for measuring a level of an acyl carnitine metabolite as an effective ingredient.

Further, according to an embodiment of the disclosure, a kit for diagnosis of oral cancer comprises the composition for diagnosis of oral cancer.

Further, according to an embodiment of the disclosure, a method of providing information for diagnosis of oral cancer comprises: (a) measuring the level of an acyl carnitine metabolite of a sample isolated from an oral cancer patient; (b) comparing the level of the acyl carnitine metabolite with the level of a control group sample; and (c) determining oral cancer when the level of the acyl carnitine metabolite of the sample isolated from the oral cancer patient is lower than the level of the control group sample.

The operation (a) comprising further measuring a level of at least one metabolite selected from the group consisting of glycerophosphorylcholine, arachidonic acid, eicosapentaenoic acid, and triglyceride.

The isolated sample comprises at least one selected from a liquid biopsy group consisting of saliva, whole blood, serum, and plasma.

Further, according to an embodiment of the disclosure, the use of acyl carnitine metabolite(s) is provided for following: a (marker) composition for oral cancer detection and/or diagnosis and a method of manufacturing the same; a kit for oral cancer detection and/or diagnosis and a method of manufacturing the same; an apparatus for oral cancer detection and/or diagnosis and a method of manufacturing the same; and a method of providing information for oral cancer detection and/or diagnosis.

### ADVANTAGEOUS EFFECTS

According to the present disclosure, oral cancer that is previously difficult to diagnose early due to the limitations of conventional tissue biopsy may be diagnosed early, and pain and risk burden of patients may be reduced using a liquid biopsy involving collection and analysis of blood.

### BRIEF DESCRIPTION OF THE DRAWINGS

FIG. 1 illustrates results of pathway analysis from The Cancer Genome Atlas (TCGA) based on RNAseq data for an oral cancer patient group and a control group.
FIG. 2 illustrates a heatmap and hierarchical cluster with Wald distance analysis for 10 metabolites in a targeted metabolite profiling approach.

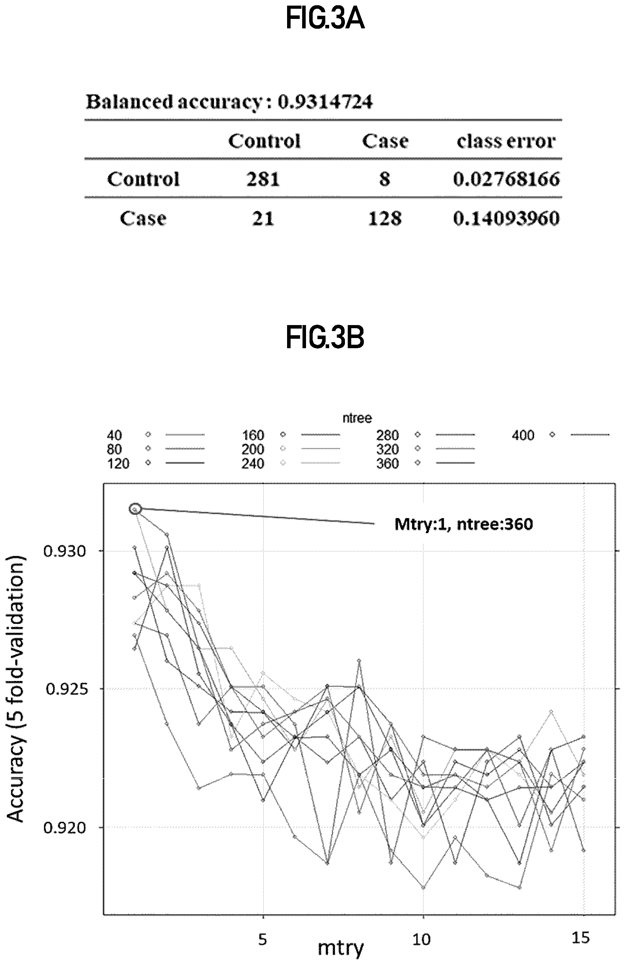

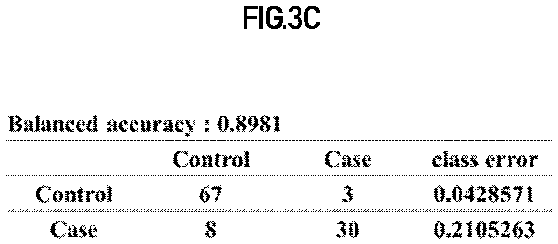

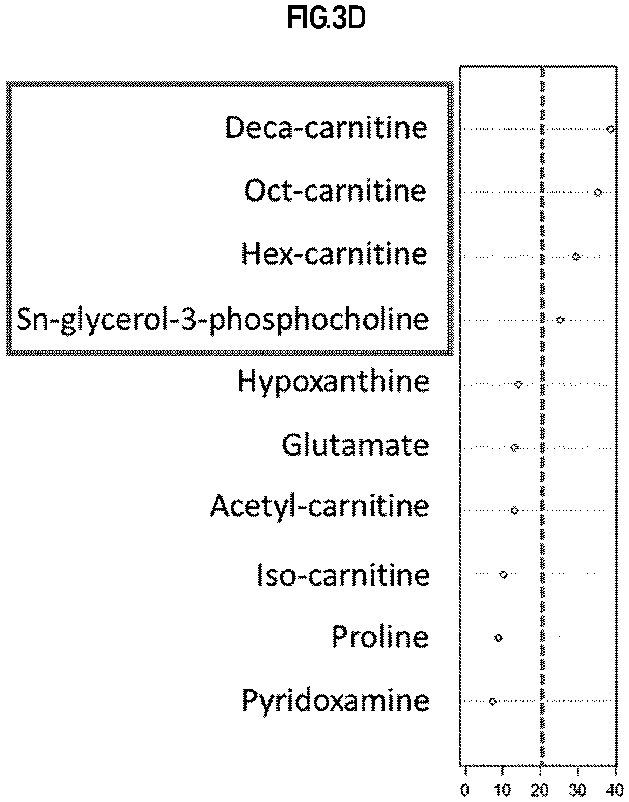

FIG. 3 illustrates results of a random forest analysis using 5-fold cross-validation in targeted metabolite profiling, and includes FIG. 3a illustrating a confusion matrix of random forest for a training set, FIG. 3b illustrating grid search results for finding optimal parameters of random forest, in which a highest accuracy point is obtained when the number of variables available for splitting at each tree node mtry is 4 and the number of trees to grow ntree is 360, FIG. 3c illustrating the confusion matrix of the random forest for a test set, and FIG. 3d illustrating 4 top metabolites using the random forest in the test set.
FIGS. 4a to 4o illustrate sensitivity, specificity, and AUC values for each of the metabolite panel identified in the validation dataset, including 4 metabolites (a-d), 2 metabolites (e-j), 3 metabolites (k-n), and 4 metabolites (o).
FIG. 5a and 5b illustrate PCA score plots obtained from UHPLC ESI Q TOF MS/MS spectra of plasma lipid compositions in positive mode and negative mode, respectively.

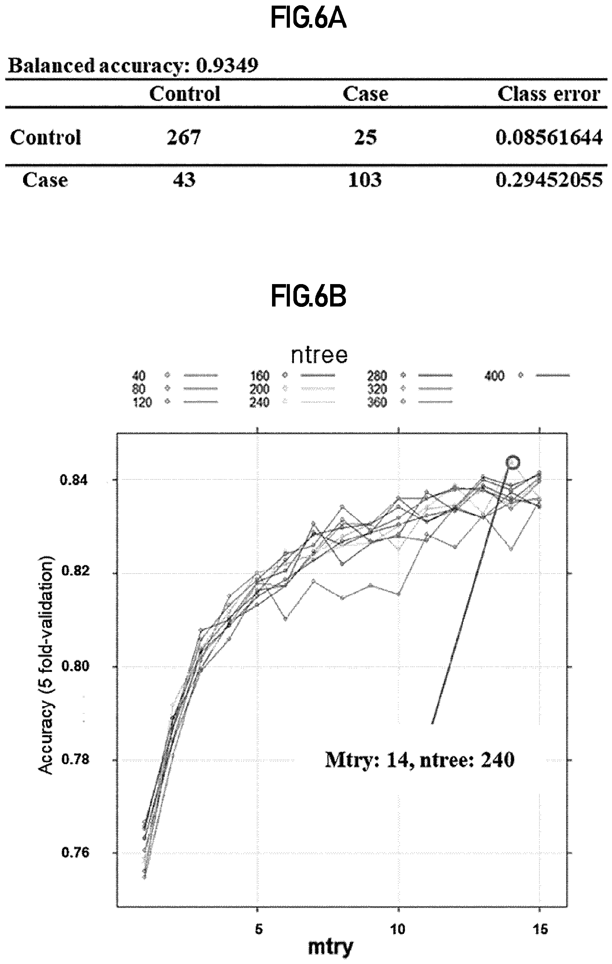

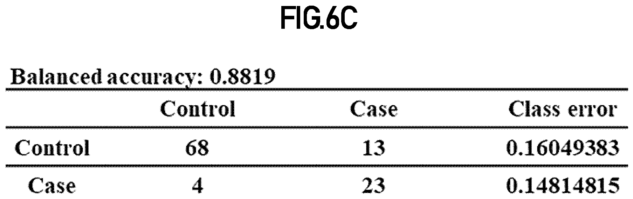

FIG. 6 illustrates results of a random forest analysis in lipid metabolite profiling with 5-fold cross-validation, and includes FIG. 6a illustrating a confusion matrix of random forest for the training set, FIG. 6b illustrating grid search results for finding optimal parameters in random forest, in which a highest accuracy point is obtained when the number of variables available for splitting at each tree node mtry is 4 and the number of trees to grow ntree is 240, FIG. 6c illustrating the confusion matrix of the random forest for the test set, and FIG. 6d illustrating 3 top metabolites using the Random Forest in the test set.

### BEST MODE FOR CARRYING OUT THE INVENTION

The present disclosure confirms that targeted and untargeted metabolite profiling based on UHPLC-ESI-Q-TOF-MS/MS was performed on samples from an oral cancer patient group and a control group, revealing that one or more acyl carnitine metabolites can be considered metabolic signals for identifying oral cancer.

In the present disclosure, the term "metabolite" refers to substances obtained from biological samples, and the biological samples from which the metabolite can be obtained may be saliva, whole blood, plasma, serum, or platelets and preferably may be plasma. In addition, the metabolite may include substances produced by metabolism and metabolic processes, substances produced by chemical metabolic reactions caused by biological enzymes and molecules, or the like.

In the present disclosure, the term "diagnosis" includes determining the susceptibility of an object to a specific disease or disorder, determining whether an object currently has a specific disease or disorder; determining the prognosis of an object with a specific disease or disorder, or therametrics, such as monitoring a status of an object to provide information on the efficacy of treatment.

According to an aspect of the present disclosure, a biomarker composition for diagnosing oral cancer includes an acyl carnitine metabolite as an effective ingredient.

In an example embodiment, oral cancer was diagnosed through the following operations: (a) extracting data based on UHPLC-ESI-Q-TOF-MS/MS from samples of an oral cancer patient group and a control group; (b) converting UHPLC-ESI-Q-TOF-MS/MS analysis results into statistically processable numerical values; and (c) statistically verifying distinctiveness of the two samples using the converted values.

In operation (b), a total of analysis time is divided into unit time intervals, and a representative value is determined as a highest numerical value of a chromatogram peak area or a height observed during each unit time. In operation (c), to compare metabolic profiling differences, machine learning analysis was performed to select metabolites representing significant differences between the two samples as a biomarker through 5-fold cross-validation, and the biomarker was analyzed and verified.

The "cross-validation" used herein refers to a method used in statistics to evaluate a model by dividing the data such that entire data is used as a test set at least once, and finding best parameters for each parameter and performance values with enhanced accuracy and reliability.

In the present disclosure, the performance value is an Area Under the Curve (AUC) and means that the higher the AUC value, the better ability to distinguish between positive and negative cases of oral cancer.

Through the 5-fold cross-validation, metabolites having an AUC value of 0.8 or more were classified as a biomarker for diagnosing oral cancer. Specifically, the biomarker includes acyl carnitine metabolites, preferably including one or more metabolites selected from the group consisting of decanoylcarnitine, octanoylcarnitine, and hexanoylcarnitine.

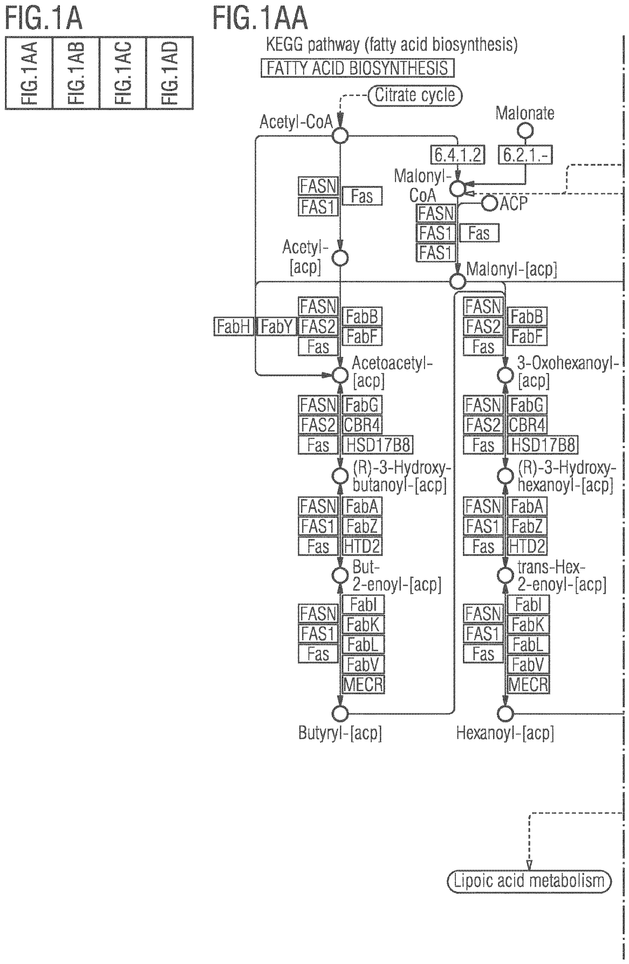

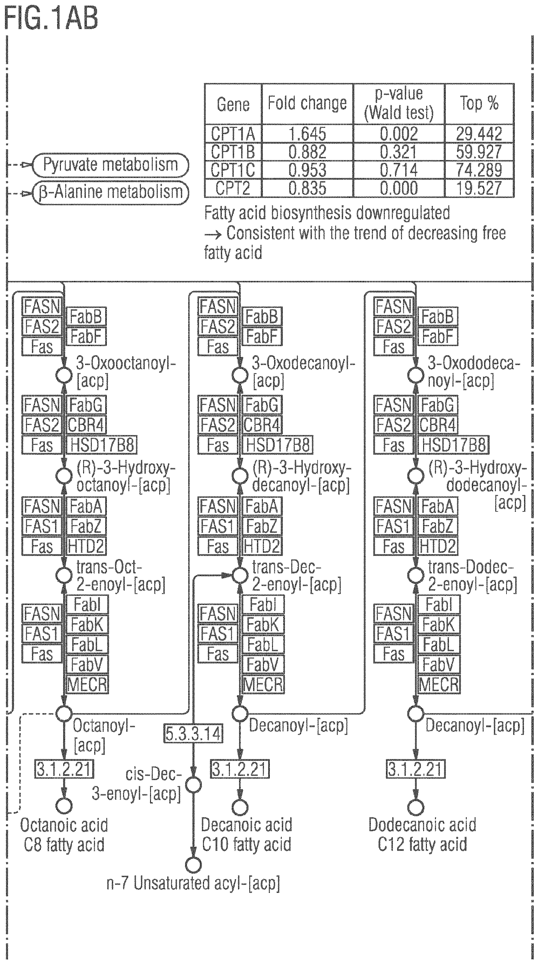

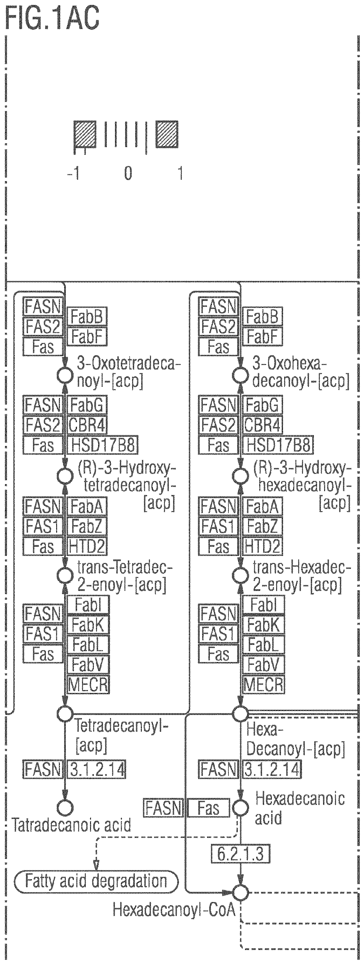

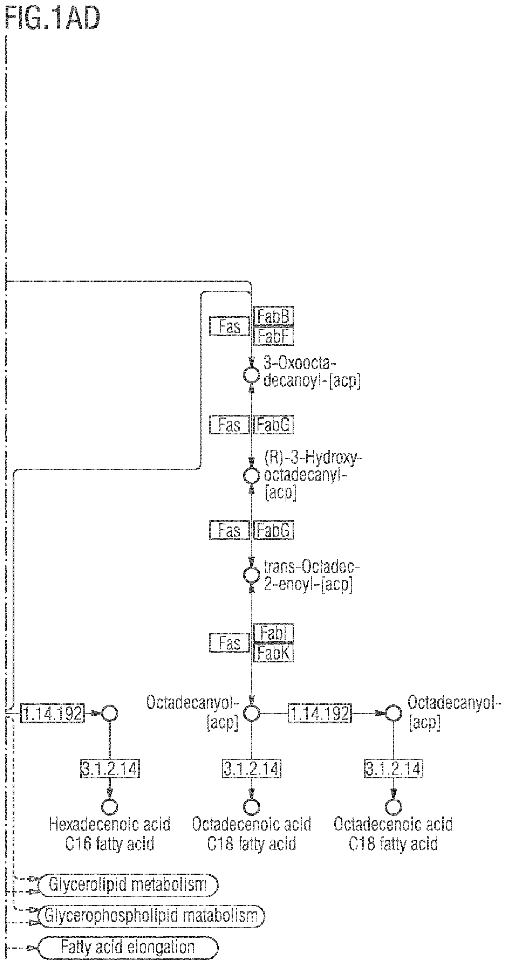

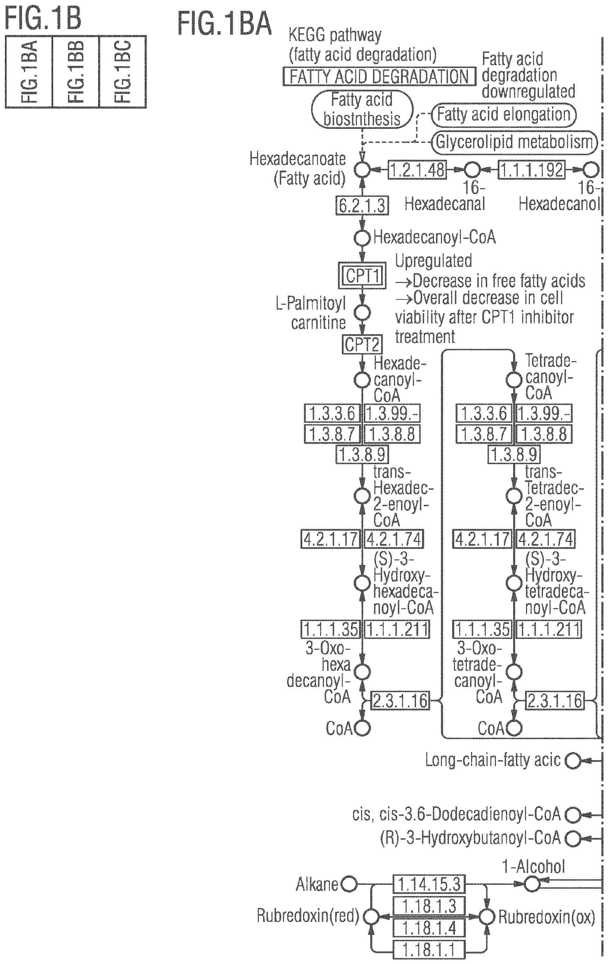

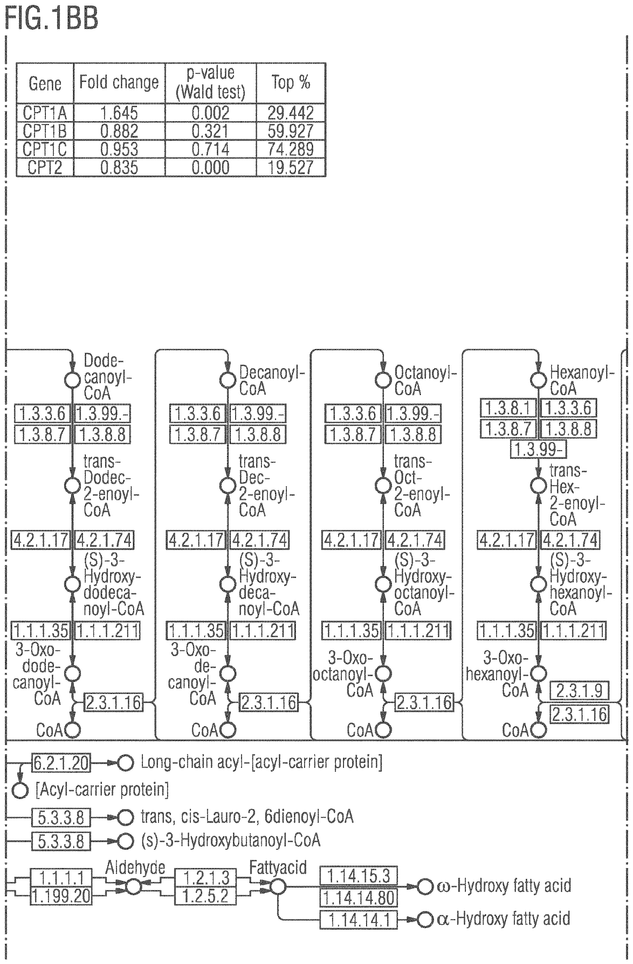

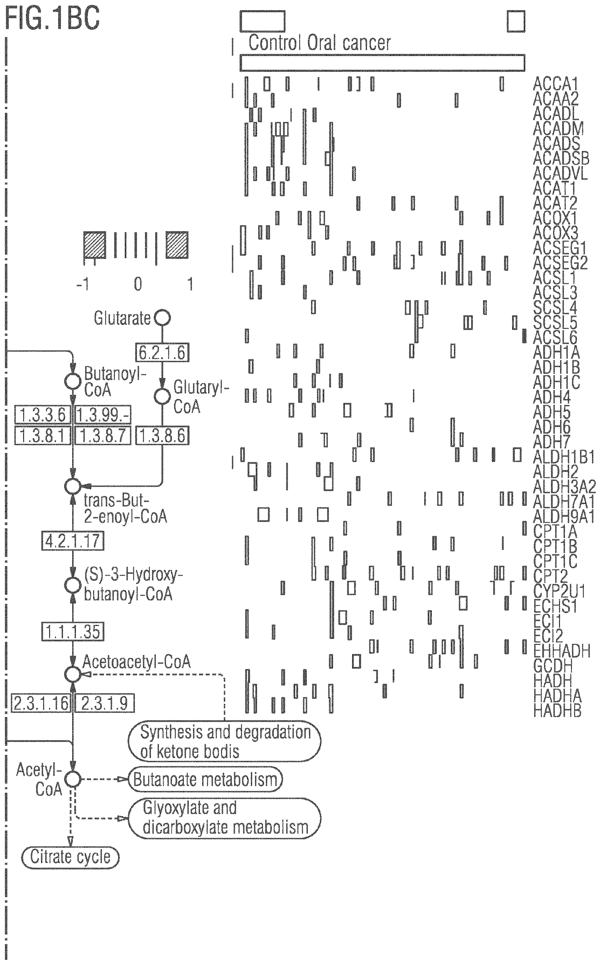

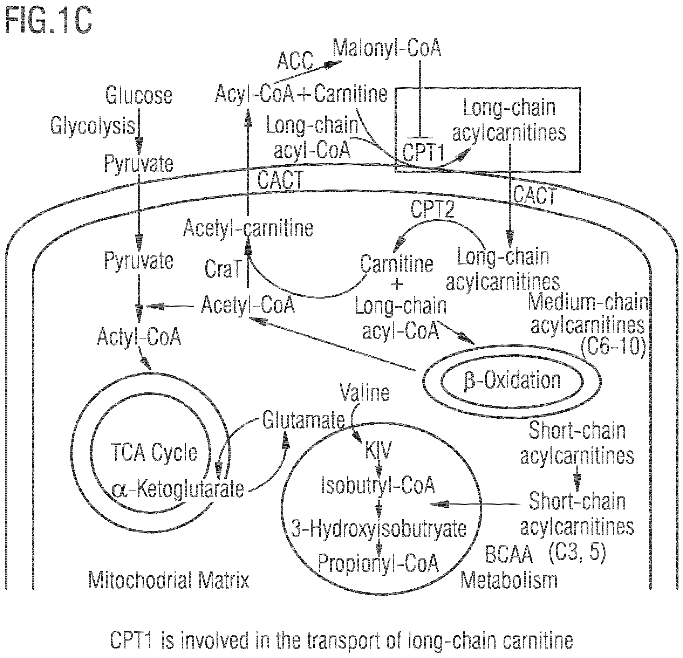

The above-mentioned acyl carnitine serves to transport fatty acids into the mitochondria during fatty acid beta-oxidation, and it has been verified as a reliable biomarker by showing a decrease in the levels of two fatty acids analyzed in lipid metabolism profiling, that is, polyunsaturated fatty acids (PUFA) arachidonic acid and eicosapentaenoic acid, in the oral cancer patient group. This is consistent with the results of the study by Mika, A., et al., which showed that the PUFA levels in the serum of colorectal cancer patients group were lower than those of healthy control group. As shown in FIG. 1, pathway analysis based on RNAseq data for oral cancer patient group and control group from the Cancer Genome Atlas (TCGA) project confirmed that the results of this study are consistent with those of the previous study.

Additionally, the biomarker composition may further include one or more metabolites selected from a group consisting of glycerophosphorylcholine, arachidonic acid, eicosapentaenoic acid, and triglyceride.

According to another aspect of the present disclosure, a composition for oral cancer includes an agent capable of measuring the level of acyl carnitine metabolites as effective ingredients.

The term "level" used herein is interchangeably used to refer to measurement values obtained using any arbitrary analysis method for detecting biomarkers in biological samples. The "level" encompasses the presence, absence, absolute quantity or concentration, relative quantity or concentration, titer, numerical values, expression values, ratios of measured values, or the like, corresponding to biomarkers in biological samples. The precise characteristics of the "level" depend on specific design and components of the analysis method used to detect the biomarker.

The term "agent" used herein refers to an agent for quantitatively detecting metabolites from biological samples, and example embodiments are not limited thereto. The agent may be primer, probe, aptamer, small molecule compound, protein, ligand, or antibody capable of complementary binding that interacts with metabolites to generate signals.

According to another aspect of the present disclosure, an oral cancer diagnostic kit includes the composition for oral cancer.

In the present disclosure, the kit essentially includes a composition for oral cancer including an agent capable of measuring the "level" of acyl carnitine metabolites as effective ingredients. Additionally, the kit may further include a component or quantification device for quantifying the agent.

The kit according to the present disclosure may be a rapid diagnostic or PCR-based diagnostic kit.

It is apparent to those skilled in the art that a kit suitable for each purpose is designed, and then implemented by appropriately modifying or changing components.

The components capable of quantifying the agent may include, but are not limited to, probes, fluorophores, chromophores, radioactive isotopes, etc., commonly used in the field of diagnostic kits.

The quantification device may be selected from a group consisting of nuclear magnetic resonance spectrometers (NMR), chromatography, and mass spectrometry, among others, for measuring the levels of each metabolite.

According to another aspect of the present disclosure, a method of providing information for diagnosis of oral cancer includes operations of (a) measuring a level of an acyl carnitine metabolite of a sample isolated from an oral cancer patient; (b) comparing the level of the acyl carnitine metabolite with a level of a control group sample; and (c) determining oral cancer when the level of the acyl carnitine metabolite of the sample isolated from the oral cancer patient is lower than the level of the control group sample.

The isolated sample may be selected from a liquid biopsy group consisting of saliva, whole blood, serum, and plasma. The operation (a) may further include measuring levels of one or more metabolites selected from the group consisting of glycerophosphorylcholine, arachidonic acid, eicosapentaenoic acid, and triglycerides.

### MODE FOR CARRYING OUT THE INVENTION

Hereinafter, preferable embodiments will be described to help understanding of the present disclosure. The following embodiments are set forth to illustrate but are not to be construed to limit the disclosure.

### Experimental Materials and Methods

### 1. Sample Collection

This study of the present disclosure was conducted with the approval of the Institutional Review Board of the National Cancer Center (IRB No. NCC2016-0147), and written consent was obtained from all study participants.

The present disclosure includes a Discovery Data Set and a Validation Data Set. The Discovery Data Set consists of 182 oral cancer patients who visited the Screening Center in the National Cancer Center Hospital and outpatient clinic from 2018 and 364 healthy people who were diagnosed with no cancer at the time of registration, matched by gender and age. The oral cancer patient group includes persons who were diagnosed with oral cancer histologically in one of the tongue, submandibular gland, upper gums, lower gums, maxillary sinus, buccal mucosa, retromolar deltoid, hard palate, soft palate, floor of mouth, and lower lip. The healthy control group excluded all people under the age of 19. The Validation Data Set included 52 oral cancer patients who have visited Seoul National University Hospital and 52 healthy individuals matched for gender with 52 oral cancer patients. The 52 healthy individuals were selected from the healthy group including the Discovery Data Set of the National Cancer Center during the same period.

Blood samples, which are specimens, were collected and then frozen immediately, and serum was separated for 20 minutes using a 3,000 rpm centrifugation at 4 degrees Celsius, and preserved at -80 degrees Celsius for further analysis.

General characteristics of participants in studies related to the present disclosure are listed in Table 1.

**[Table 1]**

| | **Discovery data set** | | | **Validation data set** | | |
|---|---|---|---|---|---|---|
| **Variable** | **Crntrol (N-364)** | **Case (N=182)** | **P-value** | **Crntrol (N=52)** | **Case (N=52)** | **P-value** |
| **Zender** | | | 1 | | | 1 |
| **Male** | 242(66.5) | 121(66.5) | | 34(65.4) | 34(65.4) | |
| **female** | 122(33.5) | 61(33.5) | | 18(34.6) | 18(34.6) | |
| **Age (years)** | 61(48.69) | 60(47.68) | 0.77 | 60.92(7.17) | 66.88(11.89) | 0.003 |
| **Age gruop** | | | | | | |
| (Age< 40) | 34(9.3) | 21(11.5) | 0.92 | 5(9.6) | 5(9.6) | <.0001 |
| (Age< 50) | 72(19.8) | 33(18.1) | | 12(23.1) | 10(19.23) | |
| (Age< 60) | 68(18.7) | 34(18.7) | | 32(61.5) | 12(23.08) | |
| (Age< 70) | 108(29.7) | 51(28.0) | | 3(5.8) | 25(4$.08) | |
| (Age ≥ 70) | 82(22.5) | 43(23.6) | | 5(9.6) | 5(9.6) | |
| **Smoking group** | | | | | | |
| Yes | 197(54.1) | 107(58.8) | 0.09 | 25(48.1) | 31(59.6) | <.001 |
| No | 97(26.6) | 75(41.2) | | 25(48.1) | 5(9.6) | |
| Unknown | 70(19.2) | 0(0.0) | | 2(3.8) | 16(30.8) | |
| **Alcohol group** | | | | | | |
| Yes | 197(54.1) | 103(56.6) | 0.03 | 16(30.8) | 19(36.5) | 0.202 |
| No | 97(26.6) | 79(43.4) | | 31(59.6) | 21(40.4) | |
| Unknown | 70(19.2) | 0(0.0) | | 5(9.6) | 12(23.1) | |

In the Discovery Data Set, 364 controls matched 182 cancer patients in terms of age and gender, so that there was no difference between groups for such variables. The results are presented as median (25%, 75%) and numbers (%), and P-values were calculated using the Kruskal-Wallis test for continuous variables and the chi-square test for categorical data. There was a significant difference in drinking status (P = 0.03) between the patient group and the control group, while there was no significant difference in smoking status (P = 0.09) between the patient group and the control group. In the Validation Data Set, significant differences were observed in age and smoking status, while there were no differences in gender and drinking status.

UHPLC-ESI-Q-TOF-MS/MS; Agilent 1290 Infinity LC and 6490 Triple Fourthpole MS system (Agilent Technologies; Palto, CA, USA) were used to separate metabolites for the Discovery Data Set and the Validation Data Set. MassHunter Workstation (Ver B.06.00, Agilent Technologies) software was used to analyze the metabolites.

### 2. LC-MS analysis method

### 2-1. Sample Preparation

(1) 50 µL of plasma sample to be used for semi-targeted profiling was extracted with 500 µL of chloroform:methanol (2:1, v/v) solution and 100 µL of water. Then, the extracted sample was dried under vacuum and centrifuged at 3000 rpm for 20 minutes. Finally, 1 µL of the dried sample was injected for use.
(2) 20 µL of plasma sample to be used for targeted profiling was extracted with 30 µL water and 150 µL acetonitrile. Then, proteins were precipitated for 1 hour and centrifuged at 4500g for 10 minutes at 4°C. Finally, an aqueous supernatant was transferred to a new 1.5 ml tube and diluted with a dilution factor of 75% CAN to add internal standards for each metabolite.
(3) 20 µL of plasma sample to be used for lipid profiling was extracted using 30 µL of water, and 250 µL of AVANTIS LIPIDOMIX, and isopropyl alcohol (1:49, v/v). Then, the extracted sample was mixed for 10 minutes and left for 2 hours to achieve complete protein precipitation. Finally, the mixed sample was centrifuged at 4500g for 10 minutes at 4°C.

### 2-2. UPLC-ESI-Q-TOF-MS/MS Analysis

(1) Semi-Targeted Profiling and Targeted Profiling: The analysis was performed using an ACQUITY UPLC (Waters, Milford, MA, USA) coupled with a Xevo TQ XS system with electrospray ionization (ESI). Separation of polar metabolites was carried out on a Scherzo SM-C18 column (2 mm x 100 mm, 3 µm; Imtakt, Kyoto, Japan) at a flow rate of 0.2 mL/min for 20 minutes. Mobile phase A consisted of 0.1% formic acid in water, and mobile phase B was consisted of 0.1% formic acid in methanol.
(2) Lipid Profiling: Chromatographic separation was performed on an ACQUITY UPLC CSH C18 (2.1 mm x 100 mm, 1.7 µm, Waters) for 20 minutes, maintaining a column temperature and flow rate of 55°C and 0.4 ml/min. In positive mode, mobile phase A was consisted of 0.1% formic acid in water: acetonitrile (40:60 v/v) and mobile phase B was consisted of 0.1% formic acid in isopropyl alcohol: acetonitrile (90:10 v/v) using a Q-TOF Micro mass detector (Waters, Milford, MA, USA). In negative mode, mobile phase A was consisted of 0.1% formic acid in water: acetonitrile (60:60 v/v) and mobile phase B was consisted of 0.1% formic acid in isopropyl alcohol: acetonitrile (90:10 v/v) using the Q-TOF Micro mass detector. For accurate mass acquisition, Lucine-enkphalin ([M + H]+: m/z 556.2771 and [M-H]-: m/z 554.2615) was used under lock-spray conditions at 5 µL/min.

### 2-3. Multi-Component Simultaneous Analysis Monitoring (dMRM)

To obtain high-quality data, retention time, internal standard concentration, etc. of the compounds were quantified using multicomponent simultaneous analysis monitoring (dMRM). QC samples were analyzed every 5 samples before sample acquisition to monitor the stability and reproducibility of the analysis system.

Table 2 shows retention times and multiple reaction monitoring transitions for metabolites selected in semi-targeted profiling. Selected reactions of decanoyl carnitine, hexanoyl carnitine, and octanoyl carnitine can be confirmed by taking ion transition values and retention time reproducibility into consideration.

**Table 2**

| Metabolite | Retention time (min) | Ionization mode | Precursor ion (m/z) | MRM ion transitions (m/z) | Collision energy (eV) | IS concentration (nM) |
|---|---|---|---|---|---|---|
| Acetyl-carnitine | 1.08 | + | 203.8 | 84.5 | 15 | 25 |
| Decanoyl carnitine | 4.86 | + | 316.2 | 84.8 | 18 | 5 |
| Glutamine | 1.18 | + | 148 | 84 | 16 | 100 |
| Hexanoyl carnitine | 4.41 | + | 260.2 | 84.5 | 15 | 5 |
| Hypoxanthine | 1.52 | + | 137.2 | 110 | 20 | 1000 |
| Isovaleryl carnitine | 4.17 | + | 246.1 | 85 | 14 | 30 |
| Octanoyl carnitine | 4.67 | + | 288.2 | 84.98 | 18 | 2.5 |
| Proline | 1.16 | + | 116 | 70 | 16 | 25 |
| Pyridoxamine | 0.88 | + | 169 | 151.8 | 10 | 100 |
| sn-glycerol 3-phosphocholine | 1.13 | + | 258 | 104 | 10 | Proline |
| Taurine | 1.14 | + | 126 | 108 | 8 | 30 |

### 3. Statistical Analysis

Multivariate statistical analysis was performed using SIMCA-P+ version 12.0 (Umetrics, Umea, Sweden). In lipid profiling, PCA was applied, and the UHPLC-ESI-Q-TOF-MS/MS dataset was scaled to unit variance before performing PCA.

Model validity was evaluated based on model parameters providing information about interpretability and predictability. Differences between groups were compared using the Kruskal-Wallis test for continuous variables and the chi-square test for categorical variables.

Metabolites obtained from global profiling were derived by achine learning analysis, including Least Absolute Shrinkage and Selection Operator (LASSO) and Random Forest (RF) methods. To avoid overfitting bias in LASSO and RF, the discovery dataset was divided into a training set (146 cases of oral cancer and 292 controls) and a test set (36 cases of oral cancer and 72 controls). The training set was utilized to build an optimistic model for metabolite selection, while the test set was employed for model validation. Metabolites were selected for further analysis by combining the results generated from both algorithms.

Subsequently, additional metabolites were selected based on fold change, multivariate adjusted conditional logistic regression analysis, and AUC values. Post-hoc multiple comparisons using the Bonferroni method were applied to identify significant differences between groups of metabolites.

Adjusted conditional logistic regression analysis was performed to estimate Odds Ratios (OR) and corresponding 95% Confidence Intervals (CI) for drinking and smoking status in the discovery dataset.

The Area Under the Curve (AUC) of the Receiver Operating Characteristic (ROC) curve was calculated to test the predictive ability of the selected metabolites. All statistical tests were set with a significance level of p > 0.05 for two-sided tests. All other statistical analyses and visualizations were conducted using the ggplot2 package on the R platform.

### <Experimental Results>

### Example 1: Polar Metabolite Profiling Analysis Results

A total of 82 metabolites were detected in plasma samples using UHPLC-ESIQ-TOF-MS/MS. To identify names, all of the metabolites were compared with actual standard references in online database in terms of accurate mass values of electrons, fragment ions, and retention times. As a result, 48 metabolites were selected for additional selection. To select the best variables distinguishing between an oral cancer patient group and a control group, a training set and a test set were constructed to derive 16 metabolites from the results of logistic regression using LASSO and 5 metabolites from the results of random forest (RF) in the 5-fold cross-validation. The accuracies of the training set and the test set for these results were respectively 98.81 % and 94.30%, and respectively 94.44% and 93.52%.

Among the 18 metabolites combined from the results of LASSO and RF in the oral cancer patient group, 6 metabolites (proline, taurine, pyridoxamine, pro-carnitine, citrulline, and hypoxanthine) increased in amount while 12 metabolites (acetylcholine, creatinine, tryptophan, aspartate, octanoyl carnitine, acetyl carnitine, iso-carnitine, glycerol phosphocholine, phenylalanine, glutamate, decanoyl carnitine, and hexanoyl carnitine) decreased in amount.

**Table 3**

| Metabolites | Fold change (Case/Control) | adjusted P-value | Conditional logistic regression OR | adjusted P-value | Train AUC | Test AUC |
|---|---|---|---|---|---|---|
| Oct-carnitine | 0.3106 | 2.80E-57 | 0.0640 | 1.26E-13 | 0.914969 | 0.949899 |
| Deca-carnitine | 0.2782 | 1.06E-50 | 0.0368 | 2.37E-11 | 0.894206 | 0.886869 |
| Sn_glycerol_3_ phosphocholine | 0.4995 | 2.08E-35 | 0.2358 | 6.24E-16 | 0.818114 | 0.852929 |
| Glutamate | 0.6612 | 3.44E-37 | 0.2342 | 2.07E-16 | 0.828862 | 0.840000 |
| Pyridoxamine | 1.3416 | 2.19E-31 | 4.5381 | 7.57E-16 | 0.805277 | 0.831919 |
| Hex-carnitine | 0.5252 | 1.65E-41 | 0.1455 | 5.96E-17 | 0.855908 | 0.821818 |
| Proline | 1.2718 | 6.01E-24 | 3.6447 | 1.57E-14 | 0.766853 | 0.762424 |
| Creatinine | 0.9208 | 2.32E-04 | 0.5624 | 1.81E-05 | 0.568521 | 0.726869 |
| Acetyl-carnitine | 0.6367 | 1.88E-27 | 0.3329 | 4.87E-14 | 0.796593 | 0.72404 |
| Hypoxanthine | 1.7927 | 1.76E-19 | 3.7789 | 1.04E-14 | 0.749081 | 0.707071 |
| Iso-carnitine | 0.6999 | 2.65E-16 | 0.4452 | 6.67E-10 | 0.715565 | 0.700202 |
| Aspartate | 0.7918 | 4.09E-15 | 0.3378 | 8.24E-12 | 0.705121 | 0.686061 |
| Taurine | 1.3407 | 9.44E-25 | 4.7644 | 1.16E-15 | 0.798678 | 0.668283 |
| Acetylcholine | 0.9534 | 1.27.E-02 | 0.7510 | 1.31E-02 | 0.547615 | 0.661414 |
| Pro-carnitine | 1.2793 | 1.58E-10 | 2.0899 | 9.75E-09 | 0.674469 | 0.600808 |
| Citrulline | 1.1605 | 8.44E-08 | 1.6324 | 2.02E-05 | 0.655886 | 0.594343 |
| Phenylalanine | 0.9095 | 6.72E-09 | 0.3930 | 4.04E-09 | 0.675406 | 0.550707 |
| Tryptophan | 0.8981 | 7.41E-05 | 0.6160 | 1.09.E-04 | 0.615467 | 0.525253 |

For the 18 metabolites, the area under the curve (AUC) of the receiver operating characteristic (ROC) was calculated to test the predictive performance for oral cancer. Ten metabolites with AUC values above 0.7 were selected (Table 3).

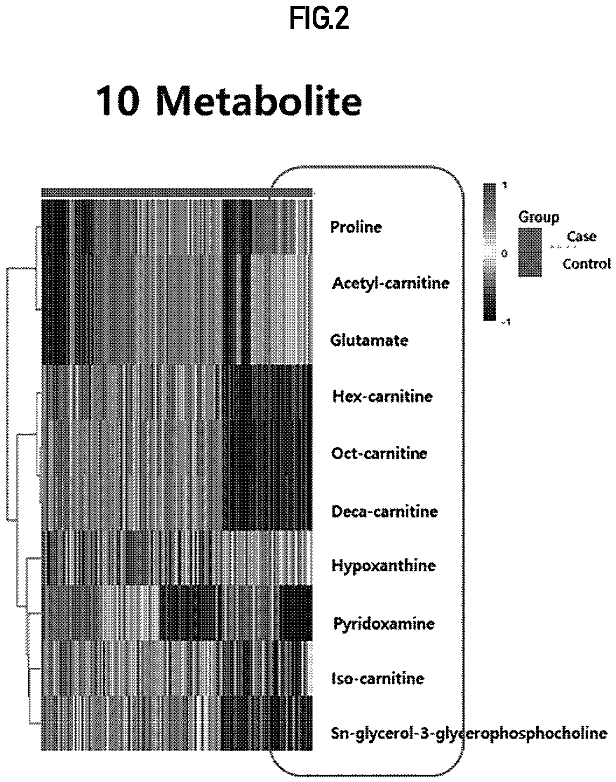

Additionally, referring to FIG. 2, a heatmap and hierarchical clustering with Wald distance analysis for the 10 metabolites revealed significant changes in octanoyl carnitine, decanoyl carnitine, hexanoyl carnitine, glycerol phosphocholine, hypoxanthine, and iso carnitine in oral cancer.

To confirm biomarker values for the above 10 metabolites, targeted profiling was performed, resulting in the identification of 6 metabolites from LASSO logistic regression and top 4 metabolites from Random Forest. The accuracies of the training set and the test set for the above results were respectively 93.25% and 93.64%, and respectively 93.14% and 89.81%.

**Table 4**

| Metabolites | Fold Change (case/control) | P-value | Conditional logistic regresion (OR) | P-value | Train AUC | Test AUC |
|---|---|---|---|---|---|---|
| Deca-carnitine | 0.515185 | 3.46E-65 | 0.073 | 2.87E-14 | 0.947129 | 0.940808 |
| Oct-carnitine | 0.489523 | 2.95E-63 | 0.0876 | 2.17E-15 | 0.940524 | 0.939192 |
| sn_glycerol_3_glycerop hosphocholine | 0.838444 | 3.15E-44 | 0.197 | 3.85E-17 | 0.85592 | 0.913131 |
| Hex-carnitine | 0.570878 | 7.77E-54 | 0.1109 | 2.24E-16 | 0.910444 | 0.884646 |
| Hypoxanthine | 1.094322 | 7.46E-13 | 2.7042 | 8.68E-12 | 0.695483 | 0.689697 |
| Iso-carnitine | 0.82226 | 5.97E-13 | 0.465 | 1.16E-09 | 0.682114 | 0.686061 |
| Acetyl-carnitine | 0.904537 | 5.48E-12 | 0.3138 | 3.18E-12 | 0.680409 | 0.650303 |
| Proline | 1.005017 | 0.391368 | 1.085 | 0.5103048 | 0.488911 | 0.531394 |

For the 8 metabolites combined from the results of LASSO and RF, the ROC curve's AUC was calculated to test the predictive performance for oral cancer, resulting in the selection of 4 metabolites with AUC values above 0.8, namely decanoyl carnitine, octanoyl carnitine, glycerol phosphocholine, and hexanoyl carnitine. Particularly, the AUC values for octanoyl carnitine and decanoyl carnitine were above 0.9 (Table 4).

Referring to FIG. 3, the results of Random Forest using 5-fold cross-validation are shown. FIG. 3A shows a confusion matrix of Random Forest for the training set, and FIG. 3B shows grid search results for finding the optimal parameters in Random Forest. A highest accuracy point is a point at which the number of variables that can be used for splitting in each tree node (mtry) is 4, and the number of trees to grow (ntree) is 360. FIG. 3C shows a confusion matrix of Random Forest for the test set, and FIG. 3D shows top 4 metabolites using Random Forest for the test set.

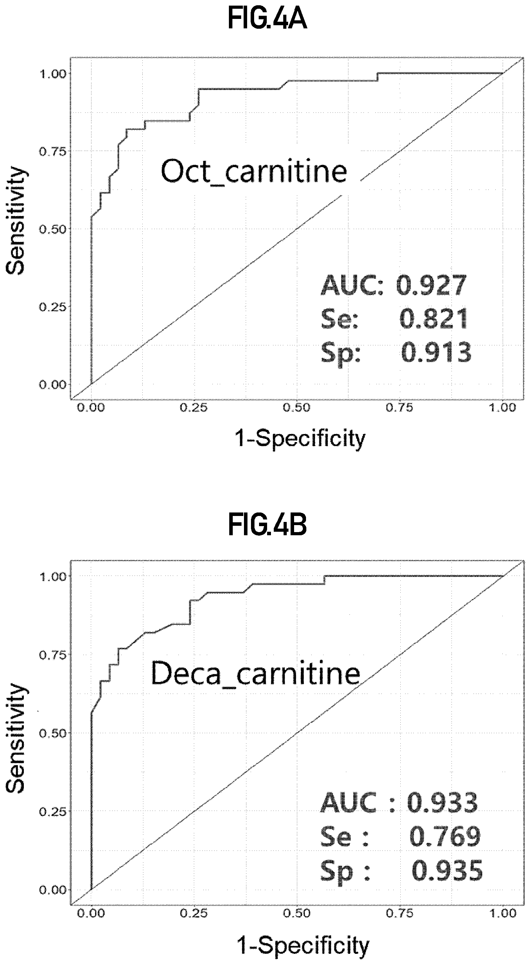

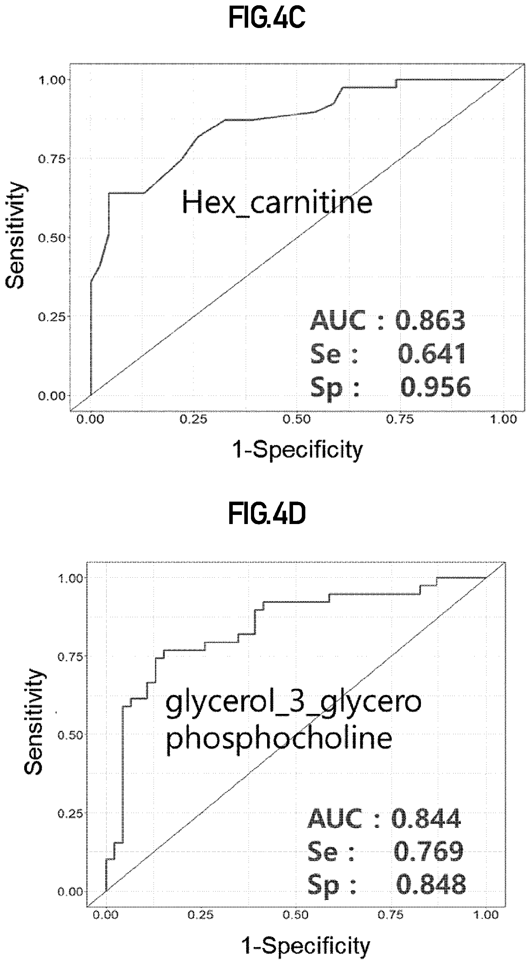

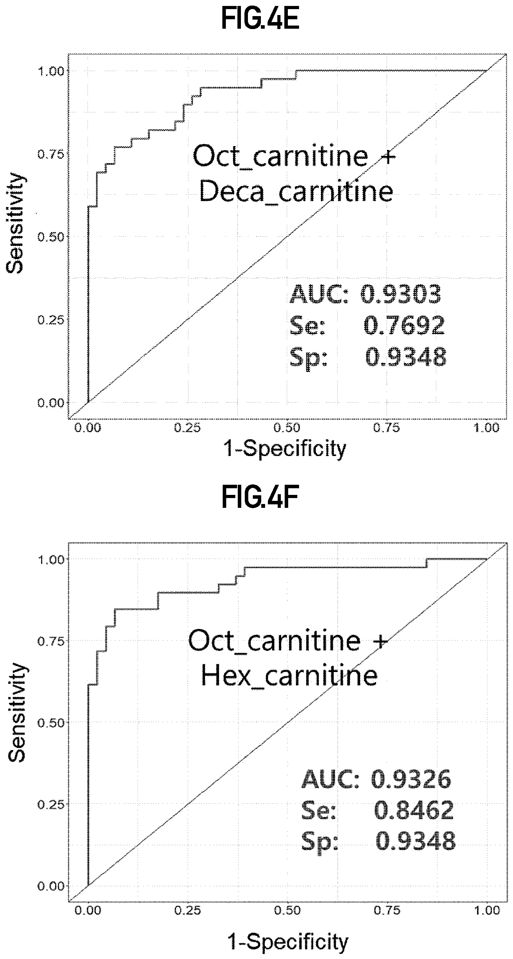

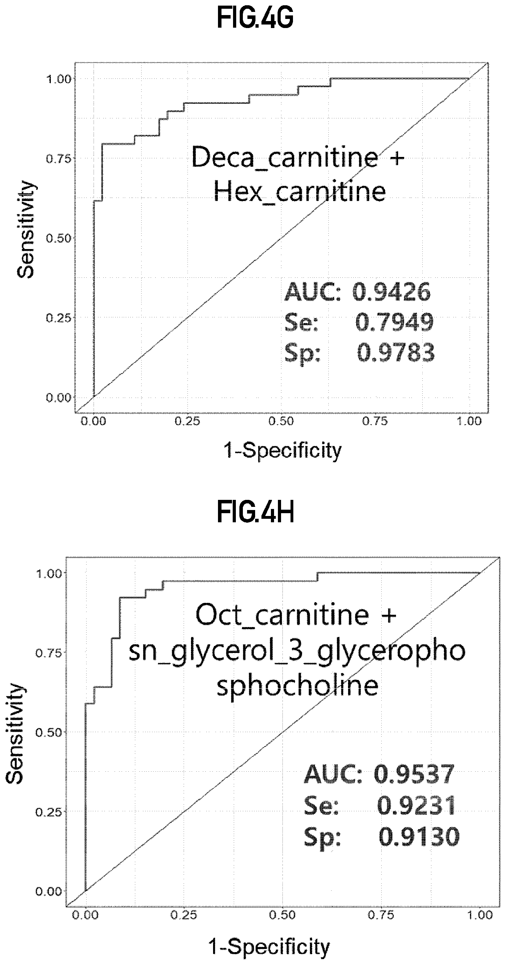

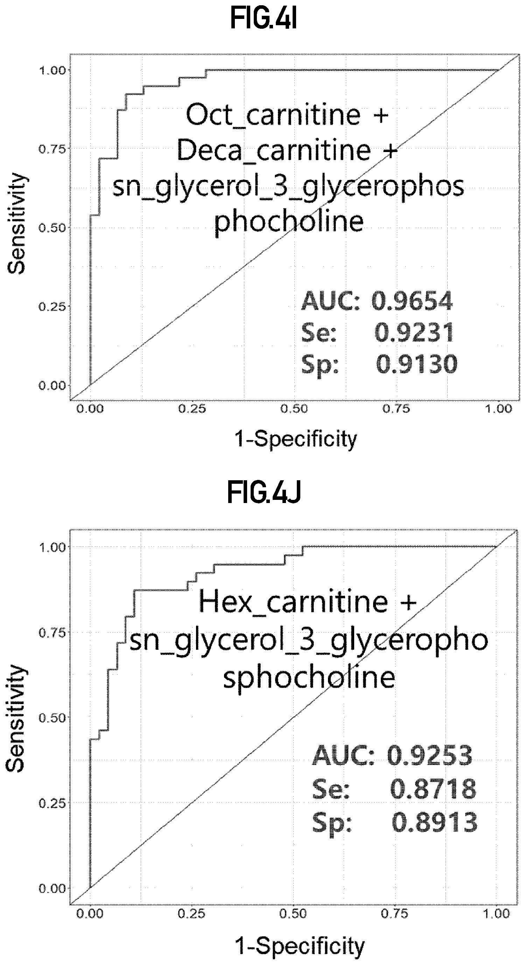

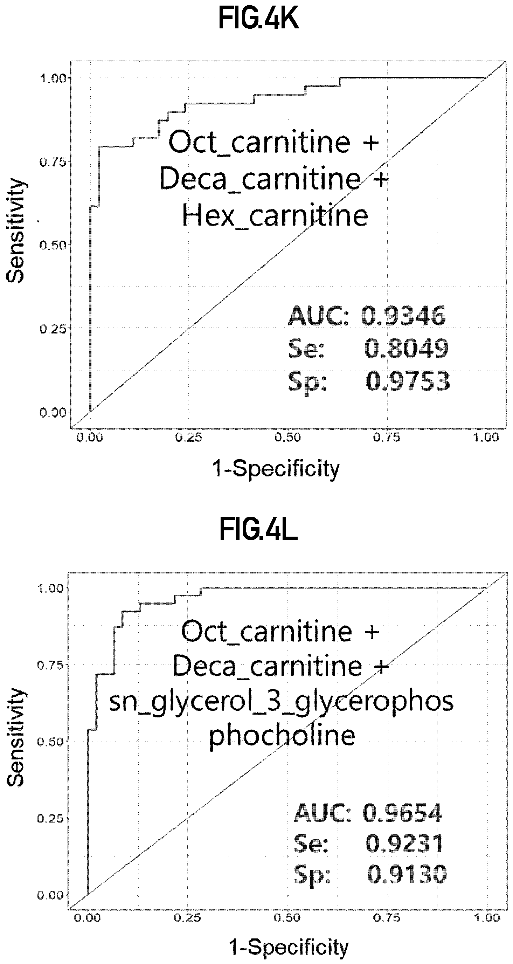

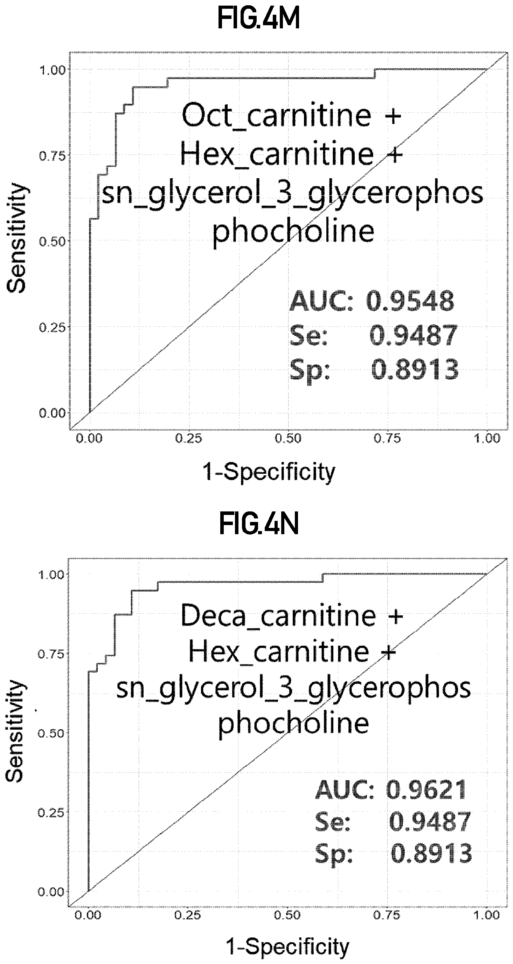

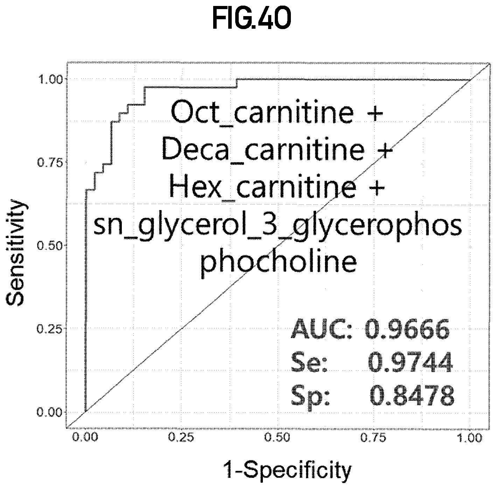

Additionally, as shown in FIGS. 4a to 4d, it can be confirmed that the AUC values of the top 4 metabolites in the validation data set are greater than 0.8. As indicated in FIG. 4o, the panel of 4 metabolites exhibits a high oral cancer identification index with a sensitivity of 0.9744, specificity of 0.8478, and an AUC of 0.9666.

### Example 2: Results of Lipid Metabolite Profiling Analysis

Among the 135 lipid metabolites detected in plasma samples using UHPLC-ESIQ-TOF-MS/MS, 86 lipid metabolites that showed significant differences between the oral cancer patient group and the control group were classified, and optimal variable values were derived through machine learning.

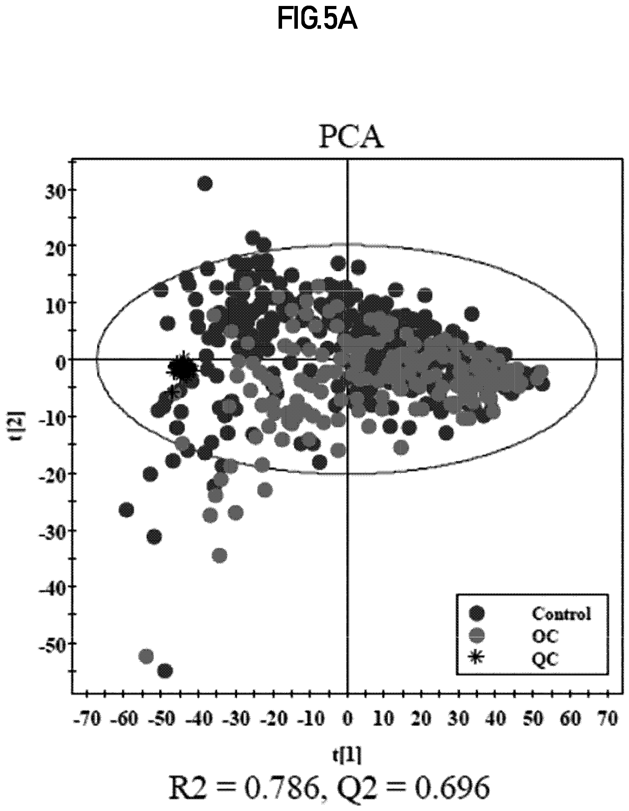

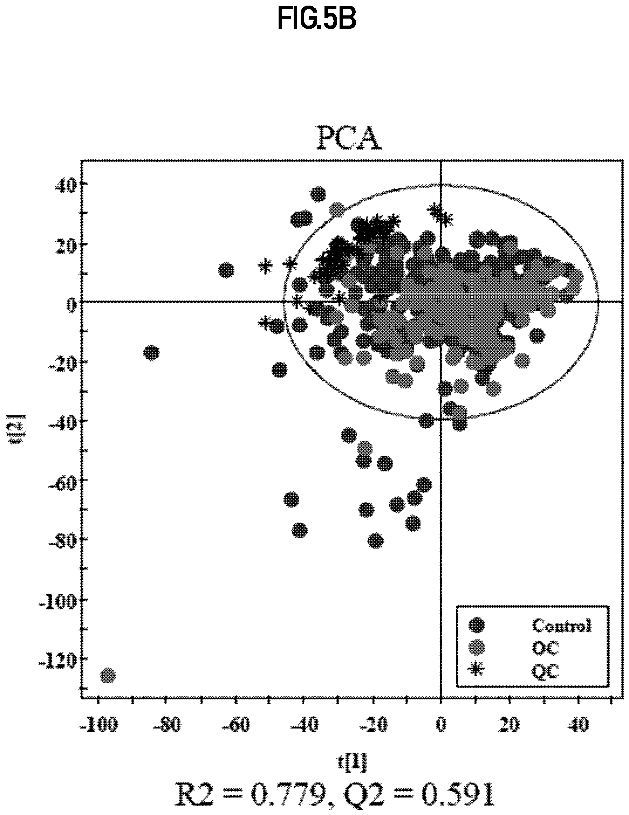

In FIG. 5, a principal component analysis (PCA) plot, where patterns between the oral cancer patient group and the control group are clearly distinguished, may be confirmed.

Lasso logistic regression resulted in the selection of 26 metabolites, and Random Forest resulted in the selection of the top 3 metabolites. The accuracies of the training set and the test set for the above results were respectively 87.33% and 79.86%, and respectively 93.49% and 88.19%.

**Table 5**

| Lipidomics | Fold change (Case/Control) | adjusted P-value | train AUC | test AUC | Conditional logistic regression OR | P-value | FDR |
|---|---|---|---|---|---|---|---|
| FFA 20:4 | 0.9011 | 0.0000 | 0.8547 | 0.8990 | 0.1630 | 0.0000 | 0.0000 |
| FFA 20:5 | 0.8800 | 0.0000 | 0.7946 | 0.7351 | 0.2150 | 0.0000 | 0.0000 |
| TG 60:13 | 0.9578 | 0.0000 | 0.7697 | 0.7270 | 0.3060 | 0.0000 | 0.0000 |
| FFA 18:2 | 0.9773 | 0.0000 | 0.6772 | 0.7633 | 0.4950 | 0.0000 | 0.0000 |
| LysoPC 18:0 | 0.9846 | 0.0000 | 0.7036 | 0.6214 | 0.3690 | 0.0000 | 0.0000 |
| PC 40:7 | 0.9723 | 0.0000 | 0.6587 | 0.5602 | 0.4050 | 0.0000 | 0.0000 |
| LysoPC 16:1 | 0.9929 | 0.0000 | 0.6804 | 0.5884 | 0.4330 | 0.0000 | 0.0000 |
| TG 44:2 | 1.0092 | 0.0000 | 0.6492 | 0.6663 | 2.0520 | 0.0000 | 0.0000 |
| FFA 20:2 | 0.9557 | 0.0000 | 0.6215 | 0.5944 | 0.6560 | 0.0013 | 0.0026 |
| TG 48:3 | 1.0245 | 0.0003 | 0.6007 | 0.6855 | 1.7790 | 0.0001 | 0.0002 |
| TG 56:7-isomer2 | 0.9861 | 0.0004 | 0.5922 | 0.5969 | 0.7500 | 0.0448 | 0.0448 |
| TG 56:9 | 0.9934 | 0.0007 | 0.5850 | 0.5795 | 0.6930 | 0.0102 | 0.0197 |
| Cer d42:1-isomer2 | 0.9850 | 0.0009 | 0.5756 | 0.6145 | 0.6230 | 0.0054 | 0.0107 |
| DG 36:4-isomer1 | 0.9962 | 0.0018 | 0.5982 | 0.6050 | 0.6920 | 0.0107 | 0.0214 |
| TG 54:2 | 1.0053 | 0.0131 | 0.5996 | 0.5891 | 1.4160 | 0.0140 | 0.0140 |
| PC 38:4 | 0.9964 | 0.0252 | 0.5687 | 0.5658 | 0.6080 | 0.0033 | 0.0066 |
| PC 38:3 | 0.9842 | 0.0253 | 0.5648 | 0.5400 | 0.6440 | 0.0110 | 0.0221 |
| DG 34:0 | 1.0017 | 0.0744 | 0.5733 | 0.5673 | 1.3480 | 0.1274 | 0.1274 |
| SMd 42:3 | 1.0006 | 0.2022 | 0.5567 | 0.5725 | 0.7880 | 0.1944 | 0.1940 |
| SMd 34:1 | 0.9990 | 0.2458 | 0.5520 | 0.5823 | 0.7670 | 0.0957 | 0.0957 |
| TG 52:3 | 0.9953 | 0.3768 | 0.5471 | 0.5575 | 0.9090 | 0.4995 | 0.4995 |
| PC 36:2 | 0.9884 | 0.4560 | 0.5631 | 0.5455 | 0.7240 | - | - |
| TG 58:8-isomer1 | 0.9974 | 0.5290 | 0.5363 | 0.5492 | 0.9270 | 0.5771 | 0.5771 |
| PC 32:2 | 1.0021 | 0.6130 | 0.5525 | 0.4638 | 1.0830 | 0.5772 | 0.5772 |
| TG 58:7-isomer2 | 0.9981 | 0.6774 | 0.5522 | 0.548 | 1.0360 | 0.7957 | 0.7957 |
| TG 52:2 | 0.9965 | 0.6956 | 0.5301 | 0.5448 | 0.9160 | 0.5399 | 0.5399 |

FIG. 6 show results of Random Forest analysis using 5-fold cross-validation. FIG. 6A shows a confusion matrix for the training set of Random Forest, and FIG. 6B shows grid search results for finding optimal parameters in Random Forest. A highest accuracy point is a point when the number of variables available for splitting at each tree node (mtry) is 14, and the number of trees to grow (ntree) is 240. FIG. 6C shows a confusion matrix for the test set of Random Forest, and FIG. 6D shows top 3 lipid metabolites using Random Forest on the test set.

As a result, when the changes in the 26 selected lipids from oral cancer patients were compared with the control group, 17 lipids had significant differences in oral cancer in terms of regression and fold change. As shown in Table 5, the fold changes of 17 lipids in the group (P value <0.05) and the AUC values of 3 lipids (FFA 20:4, FFA 20:5, and TG 60:13) that were 0.7 or more in the conditional logistic regression model analysis were selected as significant indicators for the diagnosis of oral cancer. The above-described lipids are arachidonic acid, eicosapentaenoic acid, and triglyceride.

### INDUSTRIAL APPLICABILITY

The acylcarnitine metabolites disclosed herein exhibit excellent utility as cancer diagnostic markers with the ability to distinguish oral cancer patients. Moreover, it objectively demonstrates potential as non-invasive markers for early diagnosis in oral cancer. Therefore, the composition for diagnosis of oral cancer, diagnostic kit, and methods for detecting/diagnosing oral cancer are deemed valuable.

## Claims

1. A biomarker composition for diagnosis of oral cancer, comprising an acyl carnitine metabolite as an effective ingredient.

2. The biomarker composition of Claim 1, wherein the acyl carnitine metabolite comprises at least one selected from the group consisting of decanoylcarnitine, octanoylcarnitine, and hexanoylcarnitine.

3. The biomarker composition of Claim 1, further comprising at least one metabolite selected from a group consisting of glycerophosphorylcholine, arachidonic acid, eicosapentaenoic acid, and triglyceride.

4. A composition for diagnosis of oral cancer, comprising an agent for measuring the level of an acyl carnitine metabolite as an effective ingredient.

5. A kit for diagnosis of oral cancer, comprising the composition of Claim 4.

6. A method of providing information for diagnosis of oral cancer, the method of providing information comprising:
(a) measuring a level of an acyl carnitine metabolite of a sample isolated from an oral cancer patient;
(b) comparing the level of the acyl carnitine metabolite with a level of a control group sample; and
(c) determining oral cancer when the level of the acyl carnitine metabolite of the sample isolated from the oral cancer patient is lower than the level of the control group sample.

7. The method of providing information of Claim 6, wherein the operation (a) comprising further measuring a level of at least one metabolite selected from the group consisting of glycerophosphorylcholine, arachidonic acid, eicosapentaenoic acid, and triglyceride.

8. The method of providing information of Claim 6, wherein the isolated sample comprises at least one selected from a liquid biopsy group consisting of saliva, whole blood, serum, and plasma.
